(19) Europäisches Patentamt
European Patent Office
Office européen des brevets

(11) EP 0 505 874 B1

# (12) EUROPÄISCHE PATENTSCHRIFT

(45) Veröffentlichungstag und Bekanntmachung des Hinweises auf die Patenterteilung:
**24.04.1996 Patentblatt 1996/17**

(51) Int. Cl.[6]: **C07C 17/12**, C07C 25/08

(21) Anmeldenummer: **92104481.4**

(22) Anmeldetag: **16.03.1992**

## (54) Verfahren zur Herstellung von p-Dichlorbenzol

Process for the preparation of para-dichlorobenzene

Procédé pour la préparation de para-dichlorobenzène

(84) Benannte Vertragsstaaten:
**DE FR GB IT**

(30) Priorität: **27.03.1991 DE 4110051**

(43) Veröffentlichungstag der Anmeldung:
**30.09.1992 Patentblatt 1992/40**

(73) Patentinhaber: **BAYER AG**
**D-51368 Leverkusen (DE)**

(72) Erfinder:
- **Mais, Franz-Josef, Dr.**
  **W-4000 Düsseldorf 1 (DE)**
- **Fiege, Helmut, Dr.**
  **W-5090 Leverkusen 1 (DE)**

(56) Entgegenhaltungen:
**EP-A- 0 126 669** **EP-A- 0 474 074**

- **PATENT ABSTRACTS OF JAPAN vol. 9, no. 278 (C-312)(2001) 6. November 1985**
- **PATENT ABSTRACTS OF JAPAN vol. 9, no. 293 (C-315)(2016) 20. November 1985**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.11.2/3.4

## Beschreibung

Die vorliegende Erfindung betrifft die Herstellung von p-Dichlorbenzol durch Kernchlorierung von Benzol oder Chlorbenzol in Gegenwart von Friedel-Crafts-Katalysatoren und in Gegenwart von Co-Katalysatoren in flüssiger Phase.

p-Dichlorbenzol ist ein wertvolles Zwischenprodukt zur Synthese beispielsweise von Farbstoff- und Pharmavorprodukten. Es dient weiterhin als wichtiges Monomer zur Herstellung des hochwertigen Kunststoffes Polyphenylensulfid. Besonders bei der Verwendung als Pharmavorprodukt und auch als Rohstoff für Polyphenylensulfid kommt es auf eine hohe Reinheit des p-Dichlorbenzols an. Insbesondere dürfen die isomeren Dichlorbenzole, das o-Dichlorbenzol und das m-Dichlorbenzol darin nur in sehr geringen Mengen enthalten sein.

Die Kernchlorierung von Benzol ergibt zunächst Chlorbenzol, das weiter zu einem Gemisch der drei isomeren Dichlorbenzole chloriert werden kann. Daher kann zur Herstellung von Dichlorbenzol auch von Chlorbenzol ausgegangen werden.

Im allgemeinen führt man die Chlorierung in flüssiger Phase mit gasförmigem Chlor und in Gegenwart von Friedel-Crafts-Katalysatoren, wie beispielsweise Eisen(III)chlorid, durch. So erhält man mit $FeCl_3$ als Katalysator bei 70°C Chlorierungstemperatur eine Dichlorbenzolfraktion, die aus ca. 59 % o-Dichlorbenzol, ca. 39 % p-Dichlorbenzol und ca. 2 % m-Dichlorbenzol besteht (Ullmann's Encyklopädie der technischen Chemie, 4. Aufl. 1975, Bd. 9, S. 504). Bei der destillativen Isomerentrennung erhält man aufgrund der sehr ähnlichen Siedepunkte des m- und p-Dichlorbenzols lediglich eine Auftrennung in o-Dichlorbenzol und ein Gemisch aus m- und p-Dichlorbenzol. Zur Entfernung des m-Dichlorbenzols muß ein weiterer Reinigungs- oder Trennschritt durchgeführt werden, wie z.B. Trennung an Zeolithen oder Schmelzkristallisation.

Durch zusätzliche Co-Katalysatoren zum Friedel-Crafts-Katalysator kann die Isomeren-Zusammensetzung der Dichlorbenzolmischung so verändert werden, daß ein größerer Anteil an p-Dichlorbenzol gebildet wird. Solche Co-Katalysatoren sind beispielsweise Schwefel oder Dischwefeldichlorid (Ullmann's Encyclopedia of Industrial Chemistry, 5. Ed., Vol. A6, S. 336) oder Phenothiazine, die Substituenten am N-Atom tragen (EP-A-126 669).

Die Co-Katalysatoren vermögen jedoch die Bildung des m-Isomeren nicht vollständig zu unterdrücken. So erhält man mit Schwefel als Co-Katalysator eine m-/p-Dichlorbenzolfraktion mit 0,5 bis 1,0 % m-Anteil. Mit N-Chlorcarbonylphenothiazin als Co-Katalysator (Beispiel 3, EP-A-126 669) ergibt sich bei 60°C Chlorierungstemperatur eine m-/p-Dichlorbenzolfraktion mit 0,36 % m-Anteil. Es ist somit notwendig, auch diese Gemische durch die vorgenannten Methoden auf hochreines p-Dichlorbenzol weiterzuverarbeiten.

Weiterhin ist die Chlorierung von Benzol oder Chlorbenzol in Gegenwart von bestimmten Zeolithen bekannt (EP-A-118 851, EP-A-195 514, EP-A-225 723, EP-A-231 133, EP-A-273 736, US-A-4 777 305); auch bei dieser Variante in Gegenwart von Zeolithen können weitere co-katalytisch wirkende Substanzen zugesetzt werden (EP-A-154 236, EP-A-231 662, DE-A-37 20 391, EP-A-248 931). Nachteilig an der Katalyse durch Zeolithe ist im allgemeinen der hohe Bedarf von 2 bis 6 Gew.-% an Zeolith, bezogen auf das Substrat, und weiterhin die Tatsache, daß hierbei im allgemeinen das anfallende Chlorierabgas neben HCl noch deutliche Mengen an ungenutzt entweichendem Chlor enthält. Im allgemeinen entstehen auch hier spürbare Mengen von m-Dichlorbenzol, ca. 1,0 bis 2,0 % in der m-/p-Dichlorbenzolfraktion.

Es wurde nun ein Verfahren zur Herstellung von Dichlorbenzol mit erhöhtem p-Anteil und sehr stark erniedrigtem m-Anteil durch Kernchlorierung von Benzol oder Chlorbenzol in Gegenwart von Friedel-Crafts-Katalysatoren und in Gegenwart von Co-Katalysatoren in flüssiger Phase gefunden, das dadurch gekennzeichnet ist, daß man als Co-Katalysator eine oder mehrere Substanzen der Formel (I)

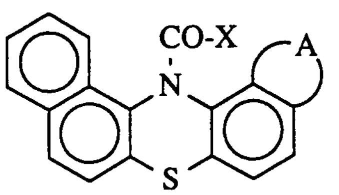

(I)

einsetzt, in der

X für Fluor, Chlor, Brom, Trifluormethyl und Pentafluorethyl

und

A für 2 Wasserstoffatome oder die Gruppe -CH=CH-CH=CH- steht.

Die erfindungsgemäß einsetzbaren Co-Katalysatoren lassen sich durch grundsätzlich bekannte Methoden, beispielsweise durch Aufschwefelung der entsprechenden Diarylamine mit Schwefel zum 1,4-Thiazinderivat und anschließende Derivatisierung am Stickstoff darstellen (Kehrmann et al., Chem. Ber. 55, 2346 (1922)).

Das erfindungsgemäße Verfahren wird in flüssiger Phase durchgeführt, wobei das Substrat Benzol oder Chlorbenzol oder ein Gemisch von ihnen auch mit einem gegen Chlor inerten Lösungsmittel, wie z.B. Methylenchlorid, Chloroform, Tetrachlorkohlenstoff, Perchlorethylen oder ähnliche, verdünnt werden kann. In bevorzugter Weise wird ohne Lösungsmittel gearbeitet. Als Chloriermittel wird Chlor gasförmig in den Reaktionsansatz eingeleitet.

Der Reaktionsdruck ist grundsätzlich unkritisch; er kann normal, erniedrigt oder erhöht sein. In bevorzugter Weise wird bei Normaldruck gearbeitet.

Als Reaktionstemperatur für die erfindungsgemäße Kernchlorierung kommt grundsätzlich der Bereich zwischen dem Erstarrungspunkt und dem Siedepunkt des Reaktionsgemisches in Frage. In bevorzugter Weise liegt die Reaktionstemperatur bei 10 bis 80°C, besonders bevorzugt bei 40 bis 70°C.

Der Wassergehalt der Reaktionsmischung ist im allgemeinen unkritisch. Es ist daher bevorzugt, alle Einsatzstoffe nicht speziell zu trocknen, sondern sie mit dem geringen Wassergehalt einzusetzen, mit dem sie üblicherweise in der chemischen Technik vorliegen. Es ist jedoch auch möglich, alle oder einzelne Einsatzstoffe zu trocknen. Im allgemeinen sollte der Wassergehalt der Einsatzstoffe nicht über den Sättigungsgrenzen bei der gewählten Reaktionstemperatur liegen. Bevorzugt liegen die Wassergehalte im Reaktionsgemisch nicht über 250 ppm, besonders bevorzugt nicht über 100 ppm.

Als Friedel-Crafts-Katalysatoren kann man die üblichen, dem Fachmann hierfür bekannten Lewis-Säuren oder Elemente, die unter den Reaktionsbedingungen Lewis-Säuren bilden, einsetzen (Ullmann's Encyclopedia of Industrial Chemistry, 5. Ed., Vol. A6, S. 343). Das sind beispielsweise die Elemente Eisen, Antimon, Aluminium, Gallium und weitere, dem Fachmann bekannte, oder deren Chalkogenide oder Halogenide, wie beispielsweise Eisen(III)-chlorid, Antimon(III)-chlorid, Aluminiumchlorid, Antimonoxychlorid, Eisensulfid und weitere. In bevorzugter Weise wird Eisen oder Eisen (III)-chlorid oder ein Gemisch von ihnen eingesetzt.

Die Mengen des erfindungsgemäß einzusetzenden Friedel-Crafts-Katalysators liegen im Bereich von 0,001 bis 1 Gew.-%, bevorzugt im Bereich von 0,01 bis 0,1 Gew.-%, bezogen auf Benzol oder Chlorbenzol oder deren Gemisch.

Die Mengen der erfindungsgemäß einzusetzenden Co-Katalysatoren liegen im Bereich von 0,001 bis 2 Gew.-%, bevorzugt im Bereich von 0,01 bis 0,5 Gew.-%, bezogen auf Benzol oder Chlorbenzol oder deren Gemisch. Dabei ist die Einsatzmenge an Co-Katalysator so zu wählen, daß das Molverhältnis von Friedel-Crafts-Katalysator und Co-Katalysator in einem Bereich von 10:1 bis 1:10 liegt. Bevorzugt wird ein Molverhältnis von 2:1 bis 1:2, besonders bevorzugt von etwa 1:1 eingestellt.

Das erfindungsgemäße Verfahren ergibt bei der Dichlorierung von Benzol bzw. der Chlorierung von Chlorbenzol oder der Chlorierung eines Gemisches beider einen besonders hohen Anteil an p-Dichlorbenzol bei gleichzeitig stark abgesenktem Gehalt an m-Dichlorbenzol. Der im Vergleich zum Stand der Technik stärker ausgeprägte p-dirigierende Effekt ist ausgesprochen überraschend, da in EP-A-126 669 (Seite 2, Zeilen 11 bis 17) ausdrücklich betont wird, daß eine zusätzliche Substitution an den Benzolringen des Phenothiazinsystems keinen weiteren wesentlichen Einfluß auf die Co-Katalyse besitzt. Die erfindungsgemäßen Co-Katalysatoren ergeben jedoch nochmals deutlich gesteigerte p-Selektivitäten.

Die erfindungsgemäße starke Absenkung des m-Gehaltes im Chloriergemisch bzw. in der Dichlorbenzolfraktion geht ebenfalls weit über den Stand der Technik hinaus. Erfindungsgemäß ist es möglich den m-Gehalt unter die GC-Nachweisgrenze von ca. 0,03 Gew.-% im Chloriergemisch zu drücken. Das bedeutet zusammen mit den hohen p-Anteilen, daß man durch Destillation direkt ein m-/p-Gemisch mit einem p-Gehalt von größer als 99,9 % erhaltenkann. Eine nachfolgende weitere Reinigung bzw. Isomerentrennung kann daher für die meisten Zwecke entfallen.

Die folgenden Beispiele erläutern die Erfindung, ohne sie jedoch auf diese einzuschränken.

Beispiel 1

Man legte 100 Gew.-Teile Benzol in einem Reaktor vor und gab 0,051 Gew.-Teile $FeCl_3$ und 0,123 Gew.-Teile des Co-Katalysators der Formel

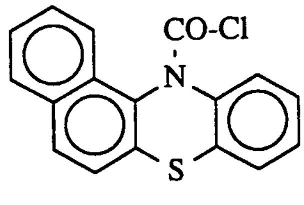

zu. Dann erhitzte man unter Rühren auf 60°C und leitete bei dieser Temperatur im Verlaufe von 4,5 h 127,5 Gew.-Teile gasförmiges Chlor gleichmäßig ein. Das Produktgemisch wurde durch flächengeeichte Gaschromatographie analysiert. Die Zusammensetzung war wie folgt:

| | |
|---|---|
| Benzol | 0,16 % |
| Chlorbenzol | 42,95 % |
| o-Dichlorbenzol | 8,22 % |
| m-Dichlorbenzol | n.n. |
| p-Dichlorbenzol | 48,67 % |
| Trichlorbenzole | n.n. |
| (n.n. bedeutet Gehalt kleiner als 0,03 %) | |

Das Verhältnis p-Dichlorbenzol zu o-Dichlorbenzol war somit p/o = 5,92 und das Verhältnis p-Dichlorbenzol zu m-Dichlorbenzol p/m ≧ 1662,3.

Ein entsprechendes Ergebnis wurde erhalten, indem man als Co-Katalysator 0,143 Gew.-Teile des Co-Katalysators der Formel

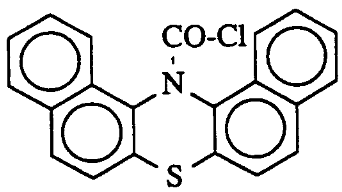

einsetzte.

Beispiel 2

Man gab 100 Gew.-Teile Chlorbenzol in einen Reaktor, gab 0,222 Gew.-Teile $FeCl_3$ und 0,57 Gew.-Teile des Co-Katalysators der Formel

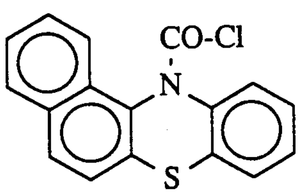

zu und thermostatisierte die Temperatur auf 20°C. Bei dieser Temperatur wurden 0,53 Gew.-Teile gasförmiges Chlor gleichmäßig im Verlaufe von 4,0 h eingeleitet. Die Zusammensetzung des Chloriergemisches war wie folgt:

| | |
|---|---|
| Benzol | 12,90 % |
| o-Dichlorbenzol | 9,46 % |
| m-Dichlorbenzol | n.n. |
| p-Dichlorbenzol | 77,64 % |
| Trichlorbenzole | n.n. |
| (n.n. bedeutet Gehalt kleiner als 0,03 %) | |

Das p/o-Verhältnis ist somit p/o= 8,20 und das p/m-Verhältnis ist p/m ≧ 2588,0.

<u>Beispiel 3</u>

Das Verfahren von Beispiel 1 wurde wiederholt, nur daß statt des dortigen Co-Katalysators 0,107 Gew.-Teile des Co-Katalysators der Formel

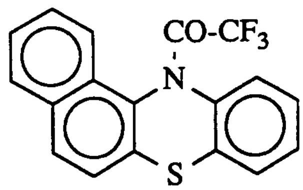

eingesetzt wurden. Die Zusammensetzung des Chloriergemisches war wie folgt:

| | |
|---|---|
| Benzol | 0,08 % |
| Chlorbenzol | 42,78 % |
| o-Dichlorbenzol | 9,88 % |
| m-Dichlorbenzol | 0,05 % |
| p-Dichlorbenzol | 47,21 % |
| Trichlorbenzole | n.n. |
| (n.n. bedeutet Gehalt kleiner als 0,03 %) | |

Das p/o-Verhältnis betrug somit p/o = 4,78 und das p/m-Verhältnis betrug p/m = 944,2 .

<u>Vergleichsbeispiel 4</u> (gemäß EP-A-126 669)

In 100 Gew.-Teilen Benzol wurden 0,050 Gew.-Teile $FeCl_3$ und 0,103 Gew.-Teile N-Chlorcarbonylphenothiazin gelöst und unter Rühren auf 60°C erhitzt. Dann wurden im Verlaufe von 4,5 h 136,7 Gew.-Teile gasförmiges Chlor gleichmäßig

eingeleitet. Die Zusammensetzung des Chloriergemisches war wie folgt:

| | |
|---|---|
| Benzol | 0,33 % |
| Chlorbenzol | 43,40 % |
| o-Dichlorbenzol | 9,79 % |
| m-Dichlorbenzol | 0,15 % |
| p-Dichlorbenzol | 46,28 % |
| Trichlorbenzole | 0,05 % |

Das p/o-Verhältnis betrug somit p/o = 4,73 und das p/m-Verhältnis betrug p/m = 308,5 .

Das Vergleichsbeispiel 4 zeigt besonders im Vergleich zu Beispiel 1 deutlich kleinere p/o- und p/m-Verhältnisse. Gleichzeitig erkennt man am Vergleich der beiden eingeleiteten Chlormengen (127,5 Gew.-Teile im Beispiel 1 und 136,7 Gew.-Teile im Vergleichsbeispiel 4), daß die erfindungsgemäße Chlorierung mit einer wesentlich besseren Chlorausbeute abläuft, da beide Gemische einen annähernd gleichen Umsatz aufweisen.

## Patentansprüche

1. Verfahren zur Herstellung von Dichlorbenzol mit erhöhtem p-Anteil und sehr stark erniedrigtem m-Anteil durch Kernchlorierung von Benzol oder Chlorbenzol in Gegenwart von Friedel-Crafts-Katalysatoren und in Gegenwart von Co-Katalysatoren in flüssiger Phase, dadurch gekennzeichnet, daß man als Co-Katalysator eine oder mehrere Substanzen der folgenden Formel

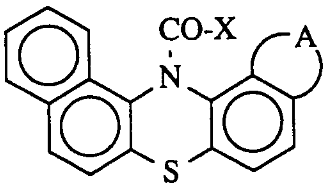

einsetzt, in der

X für Fluor, Chlor, Brom, Trifluormethyl oder Pentafluorethyl

und

A für 2 Wasserstoffatome oder die Gruppe -CH=CH-CH=CH- steht.

2. Verfahren nach Anspruch 1, dadurch gekennzeichnet daß man als Co-Katalysator eine Substanz der folgenden Formel

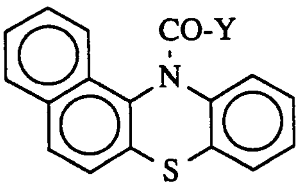

einsetzt, in der

Y für Chlor oder Trifluormethyl steht.

3. Verfahren nach Anspruch 1, dadurch gekennzeichnet, daß man als Friedel-Crafts-Katalysator Eisen oder Eisen (III)-chlorid einsetzt.

4. Verfahren nach Anspruch 1, dadurch gekennzeichnet, daß der Friedel-Crafts-Katalysator in einer Menge von 0,001 bis 2 Gew.-%, bevorzugt in einer Menge von 0,01 bis 0,2 Gew.-%, bezogen auf die Menge des Benzols oder Chlorbenzols, eingesetzt wird.

5. Verfahren nach Anspruch 1, dadurch gekennzeichnet, daß der Co-Katalysator in einer Menge von 0,001 bis 5 Gew.-%, bevorzugt in einer Menge von 0,01 bis 0,5 Gew.-%, bezogen auf die Menge an Benzol oder Chlorbenzol, eingesetzt wird.

6. Verfahren nach Anspruch 1, dadurch gekennzeichnet, daß das Molverhältnis von Friedel-Crafts-Katalysator zu Co-Katalysator wie 10:1 bis 1:10, bevorzugt 2:1 bis 1:2, besonders bevorzugt etwa 1:1 gewählt wird.

7. Verfahren nach Anspruch 1, dadurch gekennzeichnet, daß man bei einer Temperatur zwischen dem Erstarrungspunkt und dem Siedepunkt des Reaktionsgemisches, bevorzugt bei 10 bis 80°C, besonders bevorzugt bei 40 bis 70°C, arbeitet.

## Claims

1. Process for the preparation of dichlorobenzene having an increased p-content and a very greatly reduced m-content by ring chlorination of benzene or chlorobenzene in the presence of Friedel-Crafts catalysts and in the presence of co-catalysts in the liquid phase, characterised in that one or more substances of the following formula

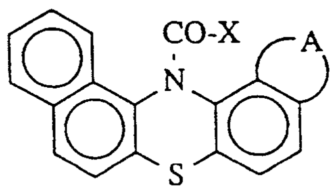

in which

X represents fluorine, chlorine, bromine, trifluoromethyl or pentafluoroethyl

and

A represents 2 hydrogen atoms or the group -CH=CH-CH=CH-

are employed as the co-catalyst.

2. Process according to Claim 1, characterised in that a substance of the following formula

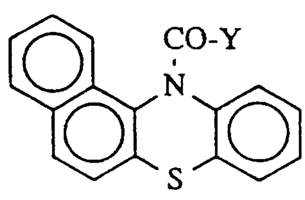

in which

Y represents chlorine or trifluoromethyl

is employed as the co-catalyst.

3. Process according to Claim 1, characterised in that iron or iron(III) chloride is employed as the Friedel-Crafts catalyst.

4. Process according to Claim 1, characterised in that Friedel-Crafts catalyst is employed in an amount of 0.001 to 2% by weight, preferably in an amount of 0.01 to 0.2% by weight, based on the amount of benzene or chlorobenzene.

5. Process according to Claim 1, characterised in that the co-catalyst is employed in an amount of 0.001 to 5% by weight, preferably in an amount of 0.01 to 0.5% by weight, based on the amount of benzene or chlorobenzene.

6. Process according to Claim 1, characterised in that the molar ratio of Friedel-Crafts catalyst to co-catalyst is chosen as 10:1 to 1:10, preferably 2:1 to 1:2, particularly preferably about 1:1.

7. Process according to Claim 1, characterised in that the reaction is carried out at a temperature between the solidification point and the boiling point of the reaction mixture, preferably at 10 to 80°C, particularly preferably at 40 to 70°C.

**Revendications**

1. Procédé de préparation d'un dichlorobenzène à teneur accrue en l'isomère p et teneur très fortement amoindrie en l'isomère m par chloruration dans le noyau du benzène ou du chlorobenzène en présence de catalyseurs de Friedel-Crafts et en présence de catalyseurs auxiliaires en phase liquide, caractérisé en ce que l'on utilise en tant que catalyseur auxiliaire une substance ou un mélange de substances de formule

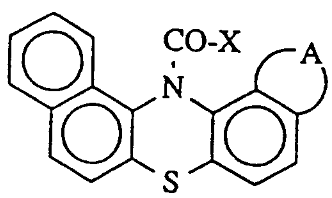

dans laquelle

X représente le fluor, le chlore, le brome, un groupe trifluorométhyle ou pentafluoréthyle et
A représente deux atomes d'hydrogène ou le groupe -CH=CH-CH=CH-.

2. Procédé selon la revendication 1, caractérisé en ce que l'on utilise en tant que catalyseur auxiliaire une substance de formule

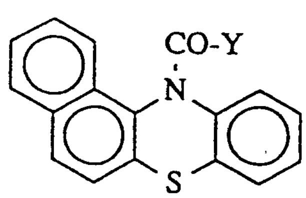

dans laquelle
Y représente le chlore ou un groupe trifluorométhyle.

3. Procédé selon la revendication 1, caractérisé en ce que l'on utilise en tant que catalyseur de Friedel-Crafts le fer ou le chlorure de fer-III.

4. Procédé selon la revendication 1, caractérisé en ce que l'on utilise le catalyseur de Friedel-Crafts en quantité de 0,001 à 2 %, de préférence de 0,01 à 0,2 %, du poids du benzène ou chlorobenzène.

5. Procédé selon la revendication 1, caractérisé en ce que l'on utilise le catalyseur auxiliaire en quantité de 0,001 à 5 %, de préférence de 0,01 à 0,5 %, du poids du benzène ou chlorobenzène.

6. Procédé selon la revendication 1, caractérisé en ce que le rapport molaire entre le catalyseur de Friedel-Crafts et le catalyseur auxiliaire est de 10 : 1 à 1 : 10, de préférence de 2 : 1 à 1 : 2 et, dans les meilleures conditions, d'environ 1 : 1.

7. Procédé selon la revendication 1, caractérisé en ce que l'on opère à une température comprise entre le point de solidification et le point d'ébullition du mélange de réaction, de préférence entre 10 et 80°C, et plus spécialement entre 40 et 70°C.